# EUROPÄISCHE PATENTSCHRIFT

(11) **EP 0 357 556 B1**
(45) Veröffentlichungstag und Bekanntmachung des Hinweises auf die Patenterteilung: **28.09.1994**
(21) Anmeldenummer: 89810628.1
(22) Anmeldetag: 24.08.1989
(51) Int. Cl.: C07D 251/52

(54) **Verfahren zur Herstellung von 6-Alkylthio-2,4-diamino-1,3,5-triazinen**
Method for the preparation of 6-alkylthio-2,4-diamino-1,3,5-triazines
Procédé pour la préparation d'alkylthio-6 diamino-2,4 triazines-1,3,5

(30) Priorität: 02.09.1988 US 240167
(43) Veröffentlichungstag der Anmeldung: 07.03.1990
(73) Patentinhaber: CIBA-GEIGY AG, 4002 Basel (CH)
(72) Erfinder: Coers, Klaus Jürgen, CH-4153 Reinach (CH)

(56) Entgegenhaltungen:
- EP-B- 0 063 327
- CH-A- 396 021
- DD-A- 80 215
- DD-A- 204 652
- US-A- 3 558 622
- CHEMICAL ABSTRACTS, Band 106, Nr. 1, 5. Januar 1987, Columbus, Ohio, USA; C. UNGVARSKY et al.: "2-alkylamino-4-chloro-6-methylthio-1,3,5-triazines", Seite 479, Spalte 1, Zusammenfassung-Nr. 5 084x
- CHEMICAL ABSTRACTS, Band 98, Nr. 5, 31. Januar 1983, Columbus, Ohio, USA; E. DRAGUSIN et al.: "2,4-Di(alkylamino)-6-alkylthio-s-triazines" Seite 652, Spalte 2, Zusammenfassung-Nr. 34 603n
- CHEMICAL ABSTRACTS, Band 77, Nr. 21, 20. November 1972, Columbus, Ohio, USA; D.H. SIM et al.: "Synthesis of prometryne. II", Seite 443, Spalte 2, Zusammenfassung-Nr. 139 990f
- CHEMICAL ABSTRACTS, Band 72, Nr. 17, 27. April 1970, Columbus, Ohio, USA; J.F. HARRIS: "Methylthiobis(alkylamino)triazines", Seite 410, Spalte 2, Zusammenfassung-Nr. 90 529t
- CHEMICAL ABSTRACTS, Band 68, Nr. 13, 25. Maerz 1968, Columbus, Ohio, USA; Z. MUELLER et al.: "Manufacturing 2-methylthio-4,6-bis(isopropylamino)-s-triazine", Seite 5769, Spalte 1, Zusammenfassung-Nr. 59 622m

## Beschreibung

Die vorliegende Erfindung betrifft ein Verfahren zur Herstellung von 6-Alkylthio-2,4-diamino-1,3,5-triazinen der Formel I 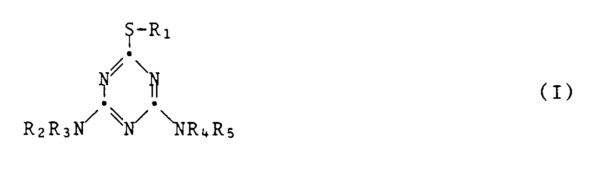
worin
R₁ C₁-C₆-Alkyl, und
R₂, R₃, R₄ und R₅ unabhänging voneinander Wasserstoff C₁-C₆-Alkyl, Allyl oder C₁-C₆-Alkoxyalkyl bedeuten, durch Umsetzung von 6-Chlor-2,4-diamino-1,3,5-triazinen der Formel II 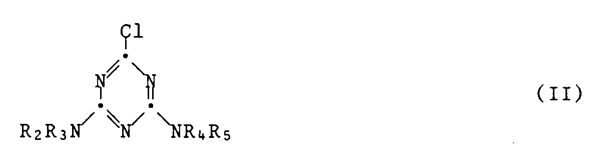
worin R₂, R₃, R₄ und R₅ die oben angegebene Bedeutung haben, mit einem Metallthioalkylat der Formel III

M(SR₁)ₙ (III)

worin R₁ die oben angegebene Bedeutung hat,
n 1 oder 2 bedeutet und
M für ein Alkalimetall steht wenn n 1 bedeutet oder für ein Erdalkalimetall steht wenn n 2 bedeutet.

Unter Alkyl ist geradkettiges oder verzweigtes Alkyl zu verstehen. Geeignete C₁-C₆-Alkylreste sind beispielsweise: Methyl, Ethyl, n-Propyl, i-Propyl n-Butyl, i-Butyl, sek.-Butyl, tert.-Butyl, n-Pentyl oder die isomeren Pentyl, Hexyl oder die isomeren Hexyl.

Beispiele für Alkoxyalkyl sind: Methoxymethyl, Methoxyethyl, Methoxypropyl, Ethoxyethyl, Ethoxymethyl oder Propyloxymethyl.

6-Alkylthio-2,4-diamino-1,3,5-triazine der Formel I können wegen ihrer wachstumshemmenden Wirkung bei Pflanzen als Herbizide verwendet werden. Besonders wertvolle Herbizide sind beispielsweise 2,4-Diisopropylamino-6-methylthio-1,3,5-triazin (Prometryn), 2-Ethylamino-4-isopropylamino-6-methylthio-1,3,5-triazin (Ametryn), 2-Isopropylamino-4-methylamino-6-methylthio-1,3,5-triazin (Desmetryn) und 2,4-Diethylamino-6-methylthio-1,3,5-triazin (Simetryn).

Es ist bereits bekannt, Ametryn durch Umsetzung von 6-Chlor-2-ethylamino-4-isopropylamino-1,3,5-triazin (Atrazin) mit Natriumthiomethylat in wässrigem Medium bei Temperaturen von 120-220°C unter einem der Reaktionstemperatur entsprechenden Druck herzustellen (US-A-3 558 622).

Dieses Verfahren ist insofern nachteilig, weil das Reaktionsgemisch selbst bei höheren Temperaturen (180°C) eine hochviskose, kaum noch rührbare Masse bildet. Ausserdem tritt bei Temperaturen von 180°C bereits in merklichem Umfang Hydrolyse auf und die Reaktion verläuft nicht mehr selektiv. Ferner ist der hohe Druck von 10-15 bar nachteilig. Eine Synthese im technischen Massstab ist daher mit hohem apparativen Aufwand verbunden.

Es ist ferner bekannt, Ametryn aus Atrazin in der Weise herzustellen, dass man Atrazin, wässriges Isopropanol (84%ig) und 50%ige wässrige Natriumhydroxidlösung vorlegt, Natriumthiomethylat zugibt, das Reaktionsgemisch anschliessend 3 Stunden unter Druck auf 80-85°C erhitzt, das Isopropanol abdestilliert und das Ametryn durch Filtration abtrennt (vgl. schweizerische Patentschrift 396.021).

Das tschechische Patent 119 837 offenbart ein Verfahren zur Herstellung von 2-Methylthio-4,6-bis(isopropylamino)-s-triazin durch Umsetzung von 2-Chloro-4,6-bis(isopropylamino)-s-triazin mit Methylmercaptan in Pyridin und Erwärmung bis 115°C in einem verschlossenen Reaktionsgefäss.

In der DD-A-80215 wird ein Verfahren zur Herstellung von 2-Methylthio-4,6-bis-alkylamino-s-triazinen durch Umsetzung von 2-Chloro-4,6-bis-alkylamino-s-triazinen mit Methylmercaptan in Gegenwart von äquivalenten Mengen wässriger Alkalihydroxidlösungen bei Temperaturen zwischen 90 und 150°C offenbart, worin die Reaktion in einem verschlossenen Gefäss stattfindet.

Das rumänische Patent RO 69 806 offenbart ein Verfahren zur Herstellung von 2,4-Di(alkylamino)-6-alkylthio-s-triazinen durch Reaktion zwischen Chloro-bis(alkylamino)-s-triazinen mit Natriumalkanthiolaten bei 140°C.

D. H. Sim und S. Y. Jo beschreiben in Choson Minjujuui Inmin Konghwaguk Kwahagwon Tongbo (koreanisch), 1, Seiten 16-20, (1972) eine Synthese von Prometryn durch Reaktion von 2-Chlor-4,6-bis(isopropylamino)-s-triazin mit NaSCH₃ in Methanol.

Das südafrikanische Patent 69 01 483 offenbart ein Verfahren zur Herstellung von Methylthio-bis(alkylamino)-triazinen durch Reaktion von einem 2-Chlor-4,6-bis(alkylamino)-s-triazin mit NaSCH₃ unter 18-stündigem Rückfluss.

Der zur Erzielung einer angemessenen Reaktionsgeschwindigkeit notwendige Ueberschuss an Metallthioalkylat beträgt bei diesen Verfahren in Bezug auf die Ausgangstriazine der Formel I üblicherweise 30 Mol %. Die Rückgewinnung bzw. Entsorgung des überschüssigen Metallthioalkylates ist mit zusätzlichem Aufwand verbunden. Bei diesen bekannten Verfahren ist ferner von Nachteil, dass die Thioalkylierung durch die höhere Oxidationsempfindlichkeit der Metallthioalkylate in organischen Lösungen unter Ausschluss von Luftsauerstoff durchgeführt werden muss, was die Prozessführung erschwert und den apparativen Aufwand erhöht.

Durch die Verwendung von Isopropanol als Lösungsmittel für die Ausgangsverbindung der Formel II ist es mit diesem bekannten Verfahren nicht möglich, 6-Alkylthio-2,4-diamino-1,3,5-triazine der Formel I ausgehend von Cyanurchlorid in einem einzigen Reaktionsgefäss herzustellen, da das als Zwischenprodukt erhaltene 6-Chlor-2,6-diamino-1,3,5-triazin für die Thioalkylierung zuerst aus seinem Reaktionsmedium - gewöhnlich Toluol - isoliert werden muss.

Mit den bekannten Verfahren lassen sich die 6-Alkylthio-2,4-diamino-1,3,5-triazine der Formel I nur mit hohem Aufwand und in wirtschaftlich unbefriedigender Weise herstellen. Es ist daher das Ziel der vorliegenden Erfindung, ein vom technischen und wirtschaftlichen Gesichtspunkt aus verbessertes und allgemein brauchbares Verfahren zur Herstellung von 6-Alkylthio-2,4-diamino-1,3,5-triazinen der Formel I bereitzustellen.

Es wurde nun gefunden, dass dieses Ziel in vorteilhafter Weise erreicht werden kann, wenn man die Umsetzung von 6-Chlor-2,4-diamino-1,3,5-triazinen der Formel II mit Metallthioalkylaten der Formel III zu einem 6-Alkylthio-2,4-diamino-1,3,5-triazin der Formel I in Gegenwart eines bestimmten tertiären Amins durchführt. Entgegen den Erwartungen zeigt sich dabei, dass die Reaktion durch die Anwesenheit des tertiären Amins bei Atmosphärendruck und niedrigeren Temperaturen unter nahezu quantitativem Umsatz des Metallthioalkylates abläuft.

Gemäss vorliegender Erfindung wird daher vorgeschlagen, 6-Alkylthio-2,4,-diamino-1,3,5-triazine der Formel I 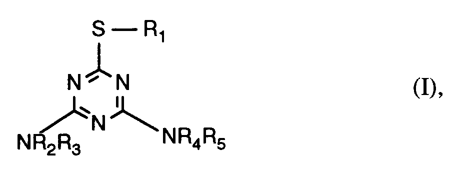
worin R₁, R₂, R₃, R₄ und R₅ die oben angegebene Bedeutung haben, in der Weise herzustellen, dass man die Umsetzung von 6-Chlor-2,4-diamino-1,3,5-triazinen der Formel II 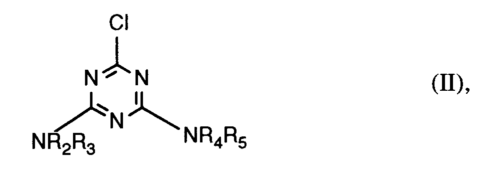
worin R₂, R₃, R₄ und R₅ die oben angegebene Bedeutung haben, mit einem geringen Überschuss eines Metallthioalkylats der Formel III

M(SR₁)ₙ (III),

worin R₁ die oben angegebene Bedeutung hat,
n 1 oder 2 bedeutet und
M für ein Alkalimetall steht, wenn n 1 bedeutet oder für ein Erdalkalimetall steht, wenn n 2 bedeutet und das Reaktionsmedium zweiphasig ist, davon eine Wasserphase und die zweite Phase ein inertes, organisches, mit Wasser nicht mischbares Lösungsmittel ist, bei Atmosphärendruck und Temperaturen zwischen 50 und 120 °C in Gegenwart eines der folgenden tertiären Amine
Chinuclidin, 1,4-Diazabicyclo[2.2.2]-octan, 1,5-Diazabicyclo[4.3.0]non-5-en (DBN), 1,8-Diazabicyclo[5.4.0]-undec-5-en (DBU), N-Methylpyrrolidin oder N,N,N',N'-Tetramethylethylendiamin
in einer Menge von 0,1 bis 10 Gew.% bezogen auf die Ausgangsverbindung der Formel II durchführt.

Bevorzugte Amine sind 1,4-Diazabicyclo[2.2.2]-octan, N-Methylpyrrolidin und N,N,N',N'-Tetramethylethylendiamin. Besonders bevorzugt ist 1,4-Diazabicyclo[2.2.2]-octan.

Die Ausgangsverbindungen der Formel II werden in üblicher Weise durch stufenweise Umsetzung von Cyanurchlorid mit den entsprechenden Aminen in einem mit Wasser nicht mischbaren Lösungsmittel, vorzugsweise Toluol, in Gegenwart einer wässrigen Lösung einer Base hergestellt. Dabei wird nach Abtrennung der wässrigen Phase eine Lösung eines 6-Chlor-2,4-diamino-1,3,5-Triazines der Formel II in einem mit Wasser nicht mischbaren Lösungsmittel erhalten, die unmittelbar für die Durchführung des erfindungsgemässen Verfahrens geeignet ist. Es ist daher vorteilhaft, die bei der Herstellung der 6-Chlor-2,4-diamino-1,3,5-triazine der Formel II erhaltene Lösung direkt für die Durchführung des erfindungsgemässen Verfahrens zu verwenden.

Die Durchführung des erfindungsgemässen Verfahrens erfolgt bei Anwesenheit von 0,1 bis 10 Gew.% an tertiärem Amin bezogen auf die Ausgangsverbindung der Formel II; bevorzugt ist eine Menge von 0,25 bis 0,35 Gew.% an tertiärem Amin bezogen auf die Ausgangsverbindung der Formel II.

Das erfindungsgemässe Verfahren wird unter Atmosphärendruck bei Temperaturen von +50°C bis +120°C, wobei Temperaturen von +75°C bis +90°C bevorzugt werden, durchgeführt.

Als inertes, organisches, mit Wasser nicht mischbares Lösungsmittel kommen offenkettige oder cyclische Kohlenwasserstoffe in Betracht, insbesondere n-Hexan, Toluol, Xylol oder Cyclohexan. Ferner sind aliphatische Ketone, insbesondere Methylethylketon oder Methylisopropylketon geeignet. Besonders bevorzugt ist Toluol.

Das Metallthioalkylat wird in geringem Ueberschuss eingesetzt. Bevorzugt ist eine Menge von 1,02 bis 1,04 Mol pro Mol Verbindung der Formel II.

In einer bevorzugten Ausführungsform des erfindungsgemässen Verfahrens wird die Umsetzung der 6-Chlor-2,4-diamino-1,3,5-triazine der Formel II mit den Metallthioalkylaten der Formel III in Gegenwart von 1,4-Diazabicyclo[2.2.2]octan bei einer Temperatur von +75°C bis +90°C in einem Reaktionsmedium aus Wasser und Toluol durchgeführt.

Eine weitere bevorzugte Ausführungsform des erfindungsgemässen Verfahrens ist jene, bei der die Thioalkylierung einer Verbindung der Formel II mit einer Menge von 1,02 bis 1,04 Mol Natriumthioalkylat pro Mol Verbindung der Formel II in Gegenwart von 0,25 bis 0,35 Gew.% 1,4-Diazabicyclo[2.2.2]octan bezogen auf die Menge der Verbindung der Formel II durchgeführt wird.

Nach der oben genannten Ausführungsform werden bevorzugt Verbindungen der Formel I, worin R₁ für Methyl, R₂ und R₄ für Wasserstoff, und R₃ und R₅ unabhängig voneinander für Methyl, Ethyl, Isopropyl oder Methoxypropyl; oder R₃ für Ethyl und R₅ für 1,2-Dimethyl-n-propyl stehen, hergestellt.

Durch das erfindungsgemässe Verfahren wird es möglich, 6-Alkylthio-2,4-diamino-1,3,5-triazine der Formel I auf besonders einfache Weise und in guten Ausbeuten herzustellen. Zur zufriedenstellenden Durchführung des erfindungsgemässen Verfahrens genügt bereits ein Ueberschuss von Metallthioalkylat von lediglich 2 bis 4 Mol % in Bezug auf das eingesetzte Triazin der Formel II. Ferner sind niedrige Reaktionstemperaturen sowie die drucklose Umsetzung der Reaktanden beim erfindungsgemässen Verfahren als vorteilhaft hervorzuheben.

Ein besonderer Vorteil des erfindungsgemässen Verfahrens besteht darin, dass man Verbindungen der Formel I ausgehend von Cyanurchlorid in einem einzigen Reaktionsgefäss in technischem Massstab herzustellen kann.

Das erfindungsgemässe Verfahren wird durch die folgenden Beispiele näher erläutert:

### Herstellungsbeispiele:

### Beispiel H 1: 2-Ethylamino-4-isopropylamino-6-methylthio-1,3,5-triazin (Ametryn) aus Atrazin

Zu einem Gemisch von 217 g (1 Mol) 6-Chlor-2-ethylamino-4-isopropylamino-1,3,5-triazin (Atrazin), 217 g Toluol, 100 g Wasser und 0,6 g 1,4-Diazabicyclo[2.2.2]octan gibt man innerhalb einer Stunde bei +80°C bis +85°C unter ständigem Rühren 147 g (1,04 Mol) 50%ige Natriumthiomethylatlösung tropfenweise hinzu. Nach 2-stündigem Rühren bei +80°C bis +85°C wird die organische Phase abgetrennt und das Toluol abdestilliert. Man erhält 223 g (98 % d. Th.) des Titelprodukts, Smp. +85°C.

Das Titelprodukt wurde gemäss Beispiel H 1 auch unter Verwendung folgender tertiärer Amine hergestellt:
a) 4 g N,N,N′,N′-Tetramethylethylendiamin
b) 1,5 g N-Methylpyrrolidin
c) 0,2 g 1,4-Diazabicyclo[2.2.2]octan (Nachrührzeit: 7 Stunden)

### Beispiel H 2: 2-Ethylamino-4-isopropylamino-6-methylthio-1,3,5-triazin (Ametryn) aus Cyanurchlorid (Eintopfverfahren)

Zu einem Gemisch von 900 g 22%iger wässriger Natriumchloridlösung und 200 g Toluol gibt man bei 0 °C bis +5°C 184,5g (1 Mol) 2,4,6-Trichlor-1,3,5-triazin (Cyanurchlorid). Zu dieser Reaktionsmischung lässt man innerhalb von 30 Minuten 59,5 g (1 Mol) Isopropylamin zutropfen. Dabei steigt die Temperatur auf +20 bis +25°C. Anschliessend wird innerhalb von 30 Minuten 80 g (1 Mol) 50%ige, wässrige Natriumhydroxidlösung tropfenweise zugesetzt. Die Temperatur lässt man während der Reaktion auf +50°C steigen. Nach Zugabe von 280 g Wasser wird der Reaktionsmischung nacheinander 45 g Ethylamin (1 Mol) und 80 g 50%ige, wässrige Natriumhydroxidlösung tropfenweise zugesetzt, wobei die Temperatur auf +75°C gesteigert wird.
Nach 30-minütigem Rühren wird die untere wässrige Phase abgetrennt. Die obere organische Phase wird mit 100 g Wasser und 0,65 g 1,4-Diazabicyclo[2.2.2]octan versetzt. Zu dieser Reaktionsmischung lässt man bei +80°C bis +85°C innerhalb einer Stunde 147 g (1,04 Mol) 50%ige Natriummethylatlösung zutropfen. Nach zweistündigem Rühren bei +80°C bis +85°C wird die organische Phase abgetrennt und das Toluol destillativ entfernt. Man erhält 223 g des Titelprodukts, Smp. +85°C.

## Patentansprüche

1. Verfahren zur Herstellung von 6-Alkylthio-2,4-diamino-1,3,5-triazinen der Formel I 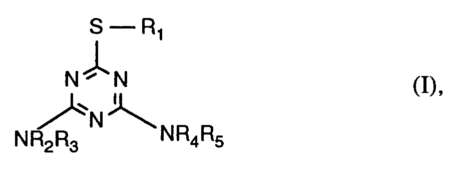 worin
R₁ C₁-C₆-Alkyl, und
R₂, R₃, R₄ und R₅ unabhängig voneinander Wasserstoff, C₁-C₆-Alkyl, Allyl oder C₁-C₆-Alkoxyalkyl bedeuten, durch Umsetzung von 6-Chlor-2,4-diamino-1,3,5-triazinen der Formel II 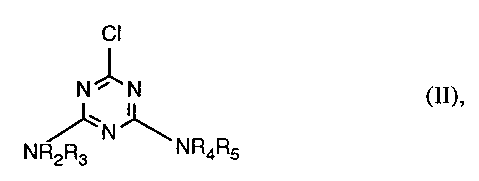 worin R₂, R₃, R₄ und R₅ die oben angegebene Bedeutung haben, mit einem geringen Überschuss eines Metallthioalkylats der Formel III
M(SR₁)ₙ (III),
worin R₁ die oben angegebene Bedeutung hat,
n 1 oder 2 bedeutet und M für ein Alkalimetall steht, wenn n 1 bedeutet oder für ein Erdalkalimetall steht, wenn n 2 bedeutet, in einem zweiphasigen Reaktionsmedium, bestehend aus einer Wasserphase und einem inerten, organischen, mit Wasser nicht mischbaren Lösungsmittel, dadurch gekennzeichnet, dass
man bei Atmosphärendruck und Temperaturen zwischen 50 und 120 °C die Umsetzung in Gegenwart eines der folgenden tertiären Amine
Chinuclidin, 1,4-Diazabicyclo[2.2.2]-octan, 1,5-Diazabicyclo[4.3.0]non-5-en (DBN), 1,8-Diazabicyclo[5.4.0]-undec-5-en (DBU), N-Methylpyrrolidin oder N,N,N',N'-Tetramethylethylendiamin
in einer Menge von 0,1 bis 10 Gew.% bezogen auf die Ausgangsverbindung der Formel II durchführt.

2. Verfahren nach Anspruch 1, dadurch gekennzeichnet, dass man als Metallthioalkylat der Formel III ein Alkalimetallthioalkylat verwendet.

3. Verfahren nach Anspruch 1, dadurch gekennzeichnet, dass man als organisches, mit Wasser nicht mischbares Lösungsmittel Toluol, Xylol, Methylethylketon, Methylisobutylketon oder Cyclohexan verwendet.

4. Verfahren nach Anspruch 1, dadurch gekennzeichnet, dass die Menge an tertiärem Amin 0,25 bis 0,35 Gew.% bezogen auf die Ausgangsverbindung der Formel II beträgt.

5. Verfahren nach Anspruch 1, dadurch gekennzeichnet, dass man als tertiäres Amin 1,4-Diazabicyclo[2.2.2]octan, N-Methylpyrrolidin oder N,N,N',N'-Tetramethylethylendiamin verwendet.

6. Verfahren nach Anspruch 1, dadurch gekennzeichnet, dass man es bei einer Temperatur von +75°C bis +90°C durchführt.

7. Verfahren nach Anspruch 1, dadurch gekennzeichnet, dass man das Metallthioalkylat in einer Menge von 1,02 bis 1,04 Mol pro Mol Ausgangsverbindung der Formel II verwendet.

8. Verfahren nach Anspruch 1, dadurch gekennzeichnet, dass man als Metallthioalkylat der Formel III ein Natriumthioalkylat verwendet.

9. Verfahren nach Anspruch 1, dadurch gekennzeichnet, dass R₂ und R₄ für Wasserstoff stehen.

10. Verfahren nach Anspruch 1, dadurch gekennzeichnet, dass man es bei einer Temperatur von +75°C bis +90°C durchführt, R₁ für Methyl, M für Natrium und R₂ und R₄ für Wasserstoff stehen.

11. Verfahren nach Anspruch 10, dadurch gekennzeichnet, dass R₃ und R₅ unabhängig voneinander Methyl, Ethyl, Isopropyl oder Methoxypropyl oder R₃ Ethyl und R₅ 1,2-Dimethyl-n-propyl bedeuten und R₂ und R₄ für Wasserstoff stehen.

12. Verfahren nach Anspruch 1, dadurch gekennzeichnet, dass man als tertiäres Amin 1,4-Diazabicyclo[2.2.2]octan verwendet.

13. Verfahren nach Anspruch 12, dadurch gekennzeichnet, dass man als Metallthioalkylat Natriumthiomethylat verwendet.

14. Verfahren nach Anspruch 1, dadurch gekennzeichnet, dass man es in Gegenwart von 0,25 bis 0,35 Gew.% 1,4-Diazabicyclo[2.2.2]octan bezogen auf die Ausgangsverbindung der Formel II und mit 1,02 bis 1,04 Mol Metallthioalkylat der Formel III pro Mol Ausgangsverbindung der Formel II bei einer Temperatur von +75°C bis +90°C in einem Medium aus Wasser und Toluol durchführt.

15. Verfahren nach Anspruch 14, dadurch gekennzeichnet, dass man als Metallthioalkylat Natriumthiomethylat verwendet.

16. Verfahren nach Anspruch 15, dadurch gekennzeichnet, dass R₂ und R₄ für Wasserstoff und R₃ und R₅ unabhängig voneinander für Methyl, Ethyl, Isopropyl oder Methoxypropyl oder R₃ für Ethyl und R₅ für 1,2-Dimethyl-n-propyl stehen.

17. Verfahren nach Anspruch 1, dadurch gekennzeichnet, dass man die bei der Herstellung der Verbindung der Formel II aus Cyanurchlorid nach Abtrennung der wässrigen Phase erhaltene Lösung aus einem 6-Chlor-2,4-diamino-1,3,5-triazin der Formel II in einem mit Wasser nicht mischbaren Lösungsmittel direkt für die Durchführung des erfindungsgemässen Verfahrens verwendet.

18. Verfahren nach Anspruch 17, dadurch gekennzeichnet, dass man als mit Wasser nicht mischbares Lösungsmittel Toluol verwendet.

## Claims

1. A process for the preparation of a 6-alkylthio-2,4-diamino-1,3,5-triazine of formula I 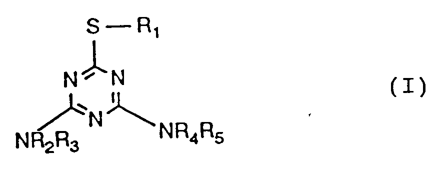 wherein R₁ is C₁-C₆alkyl, and R₂, R₃, R₄ and R₅ are each independently of the others hydrogen, C₁-C₆alkyl, allyl or C₁-C₆alkoxyalkyl, by reacting a 6-chloro-2,4-diamino-1,3,5-triazine of formula II 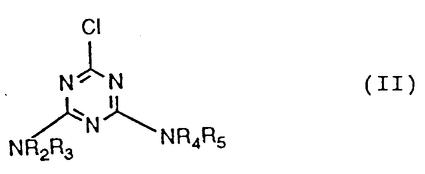 wherein R₂, R₃, R₄ and R₅ are as defined above, with a slight excess of a metal thioalkanolate of formula III
M(SR₁)ₙ (III)
wherein R₁ is as defined above, n is 1 or 2, and M is an alkali metal if n is 1 or an alkaline earth metal if n is 2, in a two-phase reaction medium consisting of a water phase and an inert, organic, water-immiscible solvent, which process comprises carrying out the reaction at atmospheric pressure and at temperatures in the range from 50 to 120°C in the presence of one of the following tertiary amines
quinuclidine, 1,4-diazabicyclo[2.2.2]octane, 1,5-diazabicyclo[4.3.0]non-5-ene (DBN), 1,8-diazabicyclo[5.4.0]-undec-5-ene (DBU), N-methylpyrrolidine or N,N,N',N'-tetramethylethylenediamine
in an amount of from 0.1 to 10 % by weight, based on the starting compound of formula II.

2. A process according to claim 1, wherein an alkali metal thioalkanolate is used as the metal thioalkanolate of formula III.

3. A process according to claim 1, wherein toluene, xylene, methyl ethyl ketone, methyl isobutyl ketone or cyclohexane is used as the organic, water-immiscible solvent.

4. A process according to claim 1, wherein the amount of tertiary amine is from 0.25 to 0.35 % by weight, based on the starting compound of formula II.

5. A process according to claim 1, wherein 1,4-diazabicyclo[2.2.2]octane, N-methylpyrrolidine or N,N,N',N'-tetramethylethylenediamine is used as the tertiary amine.

6. A process according to claim 1 which is carried out at a temperature in the range from +75°C to +90°C.

7. A process according to claim 1, wherein the metal thioalkanolate is used in an amount of from 1.02 to 1.04 mol per mol of starting compound of formula II.

8. A process according to claim 1, wherein a sodium thioalkanolate is used as the metal thioalkanolate of formula III.

9. A process according to claim 1, wherein R₂ and R₄ are hydrogen.

10. A process according to claim 1 which is carried out at a temperature in the range from +75°C to +90°C and wherein R₁ is methyl, M is sodium and R₂ and R₄ are hydrogen.

11. A process according to claim 10, wherein R₃ and R₅ are each independently of the other methyl, ethyl, isopropyl or methoxypropyl, or R₃ is ethyl and R₅ is 1,2-dimethyl-n-propyl, and R₂ and R₄ are hydrogen.

12. A process according to claim 1, wherein 1,4-diazabicyclo[2.2.2]octane is used as the tertiary amine.

13. A process according to claim 12, wherein sodium thiomethanolate is used as the metal thioalkanolate.

14. A process according to claim 1 which is carried out in the presence of from 0.25 to 0.35 % by weight of 1,4-diazabicyclo[2.2.2]octane, based on the starting compound of formula II, and with from 1.02 to 1.04 mol of metal thioalkanolate of formula III per mol of starting compound of formula II, at a temperature in the range from +75°C to +90°C in a medium consisting of water and toluene.

15. A process according to claim 14, wherein sodium thiomethanolate is used as the metal thioalkanolate.

16. A process according to claim 15, wherein R₂ and R₄ are hydrogen and R₃ and R₅ are each independently of the other methyl, ethyl, isopropyl or methoxypropyl, or R₃ is ethyl and R₅ is 1,2-dimethyl-n-propyl.

17. A process according to claim 1, wherein the solution of a 6-chloro-2,4-diamino-1,3,5-triazine of formula II in a water-immiscible solvent obtained in the preparation of the compound of formula II from cyanuric chloride after separation of the aqueous phase is used direct for carrying out the process of the invention.

18. A process according to claim 17, wherein toluene is used as the water-immiscible solvent.

## Revendications

1. Procédé pour la préparation de 6-alkylthio-2,4-diamino-1,3,5-triazines de formule I 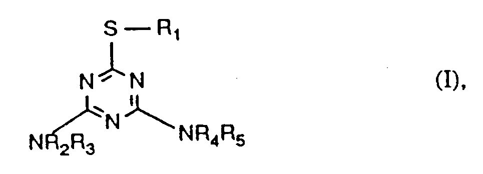 dans laquelle
R₁ représente un groupe alkyle en C₁-C₆, et
R₂, R₃, R₄ et R₅ représentent, indépendamment les uns des autres, un atome d'hydrogène ou un groupe alkyle en C₁-C₆, allyle ou alcoxy(C₁-C₆)alkyle,
par la réaction de 6-chloro-2,4-diamino-1,3,5-triazines de formule II 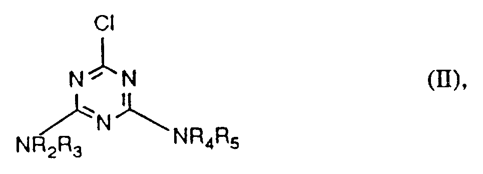 dans laquelle R₂, R₃, R₄ et R₅ ont les significations données plus haut,
avec un faible excès d'un thioalkylate métallique, de formule III
M(SR₁)ₙ (III)
dans laquelle
R₁ a la signification donnée plus haut,
n représente 1 ou 2, et
M représente un métal alcalin lorsque n représente 1, ou un métal alcalino-terreux lorsque n représente 2,
dans un milieu réactionnel en deux phases constitué d'une phase aqueuse et d'un solvant organique inerte non miscible à l'eau, caractérisé en ce que l'on effectue la réaction sous la pression atmosphérique et à des températures comprises entre 50 et 120°C, en présence de l'une des amines tertiaires suivantes:
quinuclidine, 1,4-diazabicyclo[2.2.2]octane, 1,5-diazabicyclo[4.3.0]non-5-ène (DBN), 1,8-diazabicyclo[5.4.0]-undéc-5-ène (DBU), N-méthylpyrrolidine ou N,N,N',N'-tétraméthyléthylènediamine,
en une quantité de 0,1 à 10 % en poids, par rapport au composé de départ de formule II.

2. Procédé selon la revendication 1, caractérisé en ce que, en tant que thioalkylate métallique de formule III, on utilise un thioalkylate de métal alcalin.

3. Procédé selon la revendication 1, caractérisé en ce que, en tant que solvant organique non miscible à l'eau, on utilise le toluène, le xylène, la méthyléthylcétone, la méthylisobutylcétone ou le cyclohexane.

4. Procédé selon la revendication 1, caractérisé en ce que la proportion d'amine tertiaire va de 0,25 à 0,35 % en poids, par rapport au composé de départ de formule II.

5. Procédé selon le revendication 1, caractérisé en ce que, en tant qu'amine tertiaire, on utilise le 1,4-diazabicyclo[2.2.2]octane, la N-méthylpyrrolidine ou la N,N,N',N'-tétraméthyléthylènediamine.

6. Procédé selon la revendication 1, caractérisé en ce qu'on l'effectue à une température de +75 à +90°C.

7. Procédé selon la revendication 1, caractérisé en ce que l'on utilise le thioalkylate métallique en une quantité de 1,02 à 1,04 mole par mole de composé de départ de formule II.

8. Procédé selon la revendication 1, caractérisé en ce que, en tant que thioalkylate métallique de formule III, on utilise un thioalkylate de sodium.

9. Procédé selon la revendication 1, caractérisé en ce que R₂ et R₄ représentent des atomes d'hydrogène.

10. Procédé selon la revendication 1, caractérisé en ce qu'on l'effectue à une température de +75 à +90°C, R₁ représente le groupe méthyle, M représente le sodium et R₂ et R₄ sont des atomes d'hydrogène.

11. Procédé selon la revendication 10, caractérisé en ce que R₃ et R₅ représentent, indépendamment l'un de l'autre, le groupe méthyle, éthyle, isopropyle ou méthoxypropyle, ou R₃ représente le groupe éthyle et R₅ représente le groupe 1,2-diméthyl-n-propyle, et R₂ et R₄ représentent des atomes d'hydrogène.

12. Procédé selon la revendication 1, caractérisé en ce que, en tant qu'amine tertiaire, on utilise le 1,4-diazabicyclo[2.2.2]octane.

13. Procédé selon la revendication 12, caractérisé en ce que, en tant que thioalkylate métallique, on utilise le thiométhylate de sodium.

14. Procédé selon la revendication 1, caractérisé en ce qu'on l'effectue en présence de 0,25 à 0,35 % en poids de 1,4-diazabicyclo[2.2.2]octane, par rapport au composé de départ de formule II et avec 1,02 à 1,04 mode de thioalkylate métallique de formule III, par mole de composé de départ de formule II, à une température de +75 à +90°C, dans un milieu constitué d'eau et de toluène.

15. Procédé selon la revendication 14, caractérisé en ce que, en tant que thioalkylate métallique, on utilise le thiométhylate de sodium.

16. Procédé selon la revendication 15, caractérisé en ce que R₂ et R₄ représentent des atomes d'hydrogène et R₃ et R₅ représentent, indépendamment l'un de l'autre, le groupe méthyle, éthyle, isopropyle ou méthoxypropyle, ou R₃ représente le groupe éthyle et R₅ représente le groupe 1,2-diméthyl-n-propyle.

17. Procédé selon la revendication 1, caractérisé en ce que, pour la mise en oeuvre du procédé selon l'invention, on utilise directement la solution obtenue après séparation de la phase aqueuse, lors de la préparation d'un composé de formule II à partir du chlorure de cyanuryle, qui est constituée d'une 6-chloro-2,4-diamino-1,3,5-triazine de formule II dans un solvant non miscible à l'eau.

18. Procédé selon la revendication 17, caractérisé en ce que, en tant que solvant non miscible à l'eau, on utilise le toluène.
